# EUROPEAN PATENT SPECIFICATION

(11) **EP 2 774 538 B1**
(45) Date of publication and mention of the grant of the patent: **25.05.2022**
(21) Application number: 13157968.2
(22) Date of filing: 06.03.2013
(51) Int. Cl.: A61B 5/1486, G01N 33/543, G01N 27/327, C12Q 1/00

(54) **Process for detecting an analyte using a sensor with Mn2O3 catalyst**
Verfahren zum Nachweis eines Analyten unter Verwendung eines Sensors mit Mn2O3-Katalysator
Procédé de détection d'un analyte à l'aide d'un capteur avec catalyseur Mn2O3

(43) Date of publication of application: 10.09.2014
(73) Proprietor: Roche Diabetes Care GmbH, 68305 Mannheim (DE); F. Hoffmann-La Roche AG, 4070 Basel (CH)
(72) Inventor: Koelker, Karl-Heinz, 67269 Grünstadt (DE); Staib, Arnulf, 64646 Heppenheim (DE)
(74) Representative: Altmann Stößel Dick Patentanwälte PartG mbB

(56) References cited:
- EP-A2- 0 603 154
- WO-A1-2012/130841
- US-A1- 2012 228 138
- US-B1- 6 706 473
- DATABASE WPI Week 198144 Thomson Scientific, London, GB; AN 1981-80824D XP002711292, & JP S56 119837 A (YAZAKI CORP) 19 September 1981 (1981-09-19)
- SHAN HUANG ET AL: "Glucose Biosensor Using Glucose Oxidase and Electrospun Mn2O3-Ag Nanofibers", ELECTROANALYSIS, vol. 23, no. 8, 13 August 2011 (2011-08-13), pages 1912-1920, XP055073442, ISSN: 1040-0397, DOI: 10.1002/elan.201100221
- DODEVSKA T ET AL: "Electrocatalytic reduction of hydrogen peroxide on modified graphite electrodes: application to the development of glucose biosensors", ANALYTICAL AND BIOANALYTICAL CHEMISTRY, SPRINGER, BERLIN, DE, vol. 386, no. 5, 12 September 2006 (2006-09-12), pages 1413-1418, XP019441133, ISSN: 1618-2650, DOI: 10.1007/S00216-006-0682-0

## Description

### Field of the invention

The invention relates to a process for detecting an analyte in a sample.

### Related art

Sensors and particularly biosensors are used in many technical fields to analyze a sample to determine the concentration of an analyte present in the sample and/or to determine other parameters of the sample. Technical fields can be water analysis, especially drinking water, food industry, military and medicine among others.

As used herein, the term biosensor generally refers to a sensor for detecting at least one analyte in a sample by using at least one biological detection material. As an example, the at least one biological detection material may be or may comprise at least one of an enzyme, a receptor and an antibody for the detection. Additionally, the at least one detection reaction may comprise one or more additional components, such as one or more of a catalyst, a co-enzyme, a mediator and a dye.

Thus, generally, the sensor and, more preferably, the biosensor, may comprise at least one detection material which may be adapted to perform at least one detection reaction with the analyte to be detected and/or which may be adapted to change at least one detectable property in the presence of the analyte, such as an electrical property and/or a physical properties such as a color and/or a reflectance. In the case that the sensor is a biosensor, as outlined above, the at least one detection material preferably comprises at least one enzyme, and the detection reaction preferably is or comprises an enzymatic reaction. Often, the product of such an enzymatic reaction is converted to create a detectable moiety. For this conversion, often catalysts are applied.

The use of metal oxides as catalysts for biosensors is known in the art. The catalysts accelerate the conversion reaction of H₂O₂, which is built by an analyte specific enzyme. For example, in the document EP 0 603 154 A2 and WO 2012/130841 A1, the electrode material of a biosensor comprises manganese (IV) oxide (MnO₂). Further examples of catalysts are mentioned in this document: FeOOH, Fe₃O₄, Fe₂O₃, Cr₂O₃ and V₂O₅. SHAN HUANG ET AL: "Glucose Biosensor Using Glucose Oxidase and Electrospun Mn203-Ag Nanofibers", ELECTROANALYSIS, vol. 23, no. 8, 13 August 2011 (2011-08-13), pages 1912-1920, describes a glucose sensor comprising a modified glassy carbon electrode (GCE) comprising a mixed layer of Mn₂O₃-Ag nanofibers and glucose oxidase superimposing the GCE to form a working electrode. The glucose detection is based on oxygen reduction.

The sensors are utilized with a voltage of between 350 and 500 mV. The risk of these ranges for the working voltage is an undesired reaction of further components in the sample, such as other metabolites or medicaments. Metabolites which can be oxidized in the mentioned voltage range of 350 to 500 mV are, among others, uric acid and ascorbic acid. A typical example of medicaments which can be oxidized in this voltage range is acetaminophen. The oxidation of these components at the electrode leads to an undefined increase of the electrode signal. There is no chance to distinguish the signal resulting from the analyte relevant reaction of the H₂O₂ and the signal of the undesired reaction of the further components. Thus, the sensor signal is a mixture of both reactions. As these signals cannot be separated after the detection, the signal of the analyte is overloaded by the signal reflecting the concentration of the further components. These measurements lead to a false and imprecise detection of the analyte.

The use of catalysts which lead to a reduction of the analyte in a negative voltage range is also mentioned in the prior art using hexacyanoferrate. However, this process also does not lead to reduced cross-reactions, as oxygen is reduced in the negative voltage range at about -0.1 to -0.2 V. DODEVSKA T ET AL: "Electrocatalytic reduction of hydrogen peroxide on modified graphite electrodes: application to the development of glucose biosensors",ANALYTICAL AND BIOANALYTICAL CHEMISTRY, SPRINGER, BERLIN, DE, vol. 386, no. 5, 12 September 2006 (2006-09-12), pages 1413-1418, provides methods for operating enzymatic glucose sensors in H₂O₂ reduction mode.

There is a need for sensors applicable in biological systems such as the body of a patient to detect analytes such as glucose or other metabolites in a precise and reproducible way. So far, there are known many sensors which all show a high cross reactivity with many metabolites or molecules which are often present in a living body.

### Problem to be solved

An object of the present invention is thus to reduce or even overcome at least one of the disadvantages of the state of the art.

In particular, it is an object of the present invention to provide a process for detecting an analyte which delivers precise and reproducible results.

Additionally, it is an object of the present invention to provide a process for detecting an analyte which shows a high specificity towards the analyte and little cross reactivity with other ingredients of the sample besides the analyte.

Furthermore, it is an object of the present invention to provide a process for detecting an analyte which has a high sensitivity in a broad concentration range of the analyte.

### Summary of the invention

A contribution to the solution of at least one of the above objects is provided by the subject matter of the category-forming independent claim.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

The invention is set out in the appended claim 1 directed to a process for detecting an analyte in a sample.

Providing the sensor can be achieved by any means the person skilled in the art would select for providing a sensor in a process for detecting an analyte in a sample. Providing can be any means to provide at least a part of the sensor to be contacted by a sample or a component that comprises the analyte, such as a body, especially a tissue of a body. Examples of providing the sensor are to lay the sensor on a substrate, to contact the sensor to a sample, to insert or implant the sensor into a body, and to fix the sensor to a body comprising the sample.

To lay the sensor on a substrate can be achieved by laying the sensor on a substrate such as a desk, a foil, or any other substrate in such a way that at least the working electrode can be contacted by a sample. Laying the sensor can also be achieved by fixing at least a part of the sensor to a component, such as a machine, with the aim of contacting at least the working electrode with the analyte or sample.

Contacting the sensor to a sample is preferably achieved by contacting at least a part of the sensor, especially the working electrode, with the sample. For an in-vitro sensor, the contacting can be achieved by dipping the sensor at least partly into a sample fluid. Alternatively, the sample can be applied to the sensor, especially the working electrode, via an applying means. The applying means can be selected from the group consisting of pipette, a printer, a sponge or a combination of at least two thereof. For an in-vivo sensor, the contacting can be achieved by contacting at least the working electrode of the sensor to a living body, for example the tissue of a human body.

Inserting or implanting the sensor into a body is preferably achieved by using an insertion device. The insertion device can be arranged to enter into the body of a living body. The insertion device preferably provides a tip to enter into the body of a living body. Alternatively, the sensor itself can provide a tip at one end to enter the sensor into the tissue of a living body.

Fixing the sensor to a body can be achieved by fixing the sensor to a tissue of a living body. The tissue can be inside or outside of the living body. The tissue can be selected from the group consisting of skin or mucosa. The skin can be positioned anywhere on the body of the user. The mucosa can preferably be positioned inside the nose, inside the mouth or inside the ear.

Optional dimensions, materials and further features concerning the sensor will be provided below, where the sensor is described and defined in more detail. All dimensions, materials, geometries and relations to other components described for the sensor and sensor system below are also applicable for the process.

Applying a voltage to the working electrode can be achieved by utilizing a device that is able to generate a voltage between the working electrode and a further electrode such as a counter or a reference electrode or both. In general, a power source is utilized which supplies direct voltage. The device can be selected from the group consisting of a potentiostat, a battery, an accumulator and a power supply unit or a combination of at least two thereof. Preferably, a potentionstat is utilized to supply the voltage to the working electrode. To realize the application of a voltage to the working electrode, the power source can be connected to the contact leads of the sensor, which are described in detail below for the sensor. The voltage applied to the working electrode can be regulated by a voltage regulator. When contacting the working electrode, to which a voltage is applied, with the sample, H₂O₂ molecules in the sample will react with the working electrode. The reaction of the H₂O₂ molecules at the working electrode provokes a current flow in the sensor which can be measured by a detection device. The measured current is preferably proportional to the amount of H₂O₂ in the sample. In at least a specific voltage range, the reaction rate of the H₂O₂ molecules at the working electrode is directly dependent on the diffusion rate of the H₂O₂ molecules in the sample. That leads to diffusion controlled measurement of the H₂O₂ molecules and to a specific current measured for a specific H₂O₂ concentration, also called a diffusion threshold current. Thus, the measured current signal alters proportional to a H₂O₂ concentration change in this diffusion controlled voltage range. An increase or a decrease of the voltage applied to the working electrode will not alter the reaction rate of the H₂O₂ molecules in this specific diffusion controlled voltage range.

The voltage of the working electrode is in a range from 50 to 150 mV if compared to a reference electrode comprising Ag/AgCl with 3 m KCl. The counter electrode, the reference electrode or both can comprise graphite. The reference or the counter electrode can have a size in a range from 0.1 to 10 mm², or in a range of from 0.5 to 5 mm², or in a range from 1 to 2 mm². The counter electrode can have a size, where an additional reference electrode is not necessary. For example, the counter electrode has a size for which the potential does not change more than 10 mV during an operation of the sensor measuring currents. Preferably, the sensor measures currents in a range from -100 nA to 100 nA, or in a range from -50 nA to 50 nA, or in a range from -20 nA to 20 nA.

As used herein, a sensor is a device that is able to detect at least one analyte in a sample. For this purpose, the sensor may be adapted to generate a detectable signal and/or to change at least one detectable property when coming into contact with the analyte. As used herein, an analyte is a specific compound or combination of compounds to be detected. The detectable signal correlates with the concentration of the analyte in a reproducible way.

The sensor is an electrochemical sensor for detecting the at least one analyte. As used herein, the term electrochemical sensor generally refers to a sensor having at least two sensor electrodes and which is adapted to detect the concentration of the at least one analyte based on at least one measurement of a current .

The at least two sensor electrodes comprises at least one working electrode, the electrical potential of which varies in accordance with the concentration of the analytes to be detected, and, further, at least one further element, such as at least one reference electrode and/or at least one counter electrode. The working electrode comprises a plurality of detection materials. The detection material may be part of the first electrode material, may form the first electrode material, or the first electrode material may be part of the detection material. A current is measured and used as a measure of the concentration of the analyte.

The sensor can have any shape that the person skilled in the art would consider suitable for use in the context of the present invention. As an example, the sensor may be or may comprise one of a strip-shaped sensor, a sensor disk, a sensor tape, a sensor bar and a sensor needle. The sensor may comprise one or more test fields, wherein each test field comprises the detection materials.

The sensor comprises a substrate. The substrate can be any substrate that the person skilled in the art would consider suitable for use in the context of the present invention. The substrate is one of the form building parts of the sensor, so if not specified differently the specifications for the sensors shape and dimensions also apply for the substrate. The substrate and preferably also the sensor can have a shape at least in two dimensions, selected from the group consisting of round, oval, angular or a mixture of at least two thereof. The substrate or the sensor can furthermore have a shape selected from the group consisting of a plate, a strip, a tape, a cube, a cuboid, a cone, a ball, a pyramid, a disc, a needle or a mixture of at least two thereof. In a preferred embodiment of the substrate or the sensor, the substrate or the sensor has the shape of a needle or bar.

The dimensions of the sensor can be any one the person skilled in the art would consider suitable for a sensor for the detection of an analyte. The sensor can for example have a length in a range of from 0.1 to 30 cm, or in the range of from 1 to 20 cm, or in a range of from 2 to 10 cm. The width of the sensor can be in a range of from 0.1 to 50 mm, or in a range of from 0.5 to 20 mm, or in a range of from 1 to 10 mm. The height of the sensor can be in a range of from 0.01 to 100 mm, or in a range of from 0.05 to 10 mm, or in a range of from 0.1 to 5 mm. As the substrate is a main form building part of the sensor, the dimensions mentioned for the sensor also apply for the dimensions of the substrate.

The substrate can comprise at least one material that enables the substrate to be superimposed by at least one further material. The substrate can be a solid. The substrate can be flexible or rigid. Preferably, the substrate is flexible, which means that the substrate may be deformed manually in at least one direction, such as by forces of less than 10 N. The material of the substrate can be selected from the group consisting of a glass, a polymer, a ceramic, a paper, a metal oxide and a metal or a combination of at least two thereof. As an example of a multi-component substrate, the substrate may be or may comprise a multi-layer setup having two or more layers, such as a laminate. In one embodiment of the sensor, the material of the substrate comprises a polymer. The polymer is preferably selected from the group consisting of a polyethylene, a polypropylene, a polystyrene, a polyester and a polyimide or a combination of at least two thereof. The polymer can have a molecular weight in a range of from 1 000 to 1 000 000 g/mol, or in a range of from 5 000 to 500 000 g/mol, or in a range of from 10 000 to 100 000 g/mol. Preferably, the polymer is selected from the group of a polyimide, for example polybismaleimide (PBMI), polybenzimidazole (PBI), polyoxadiazobenzimidazole (PBO), polyimidesulfone (PISO) and polymethacrylimide (PMI), or a mixture of at least two thereof. The substrate can comprise a polymer in a range from 10 to 100 wt.-%, or in a range of from 20 to 95 wt.-%, or in a range of from 30 to 90 wt.-%. The mentioned components of the substrate may add up to 100 wt.-%.

The substrate can have any form or geometry that is suitable for use in a sensor. The substrate preferably has the form or geometry as described for the sensor above. The thickness or diameter of the substrate can lie in a range of from 0.1 to 10 mm, or in a range of from 0.5 to 5 mm, or preferably in a range of from 1 to 3 mm. In the case of a cubical or cuboid extension of the substrate, the substrate can have a horizontal extension, defined as the product of the width and the length, in a range of from 1 mm² to 100 cm², or in a range of from 10 mm² to 50 cm², or in a range of from 50 mm² to 10 cm². Besides a symmetrical shape, the sensor or the substrate can have an asymmetrical form. For example, the substrate can be L-shaped. The extensions of the longer part of the L can be in the ranges for the rectangular substrate as described before.

The substrate comprises a sensor surface. The sensor surface can be defined as the part of the surface of the substrate that will be superimposed by the conductive material or the electrode material. As outlined above, the electrode material may form a detection material and/or may be part of a detection material. Therein, the conductive material and/or the electrode material may be in direct contact with the substrate. Alternatively, at least one intermediate layer may be interposed between the conductive material and/or the electrode material, such as an insulation layer and/or a diffusion barrier.

The sensor surface can have an area that lies in a range of from 5 to 90 %, or in a range of from 10 to 80 %, or in a range of from 20 to 70 % of the whole surface area of the substrate. The sensor surface of the sensor can be provided on any side or surface of the substrate. The sensor surface can have any extension of the substrate that is accessible or contactable, at least during the production process, from the outside of the sensor or substrate. Any accessible or contactable surface of the substrate can also be named as outer surface. The extension of at least one surface of the sensor can be uniform or consistent in its shape. The extension of a surface can be even, tilted or curved. The side or surface on which the sensor surface is provided on the substrate can be positioned on the outside of the substrate of the sensor. The sensor surface can be the whole or at least a part of the outer surface of the substrate. The sensor surface can have any shape which the at least one surface of the substrate provides. The shape of the sensor surface can be selected from the group consisting of even, tilted, angular and curved or a mixture of at least two thereof.

The substrate may have a cuboid extension, wherein the sensor surface may be provided on at least one of the broader sides of the substrate. The sensor surface can also be provided on one of the smaller sides of the cuboid. The sensor surface can furthermore be provided on more than one surface of the substrate. The sensor surface can extend on two or more surfaces of the substrate. The sensor surface can have an extension in a range of from 0.05 to 50 cm², or in a range of from 0.1 to 20 cm², or in a range of from 0.2 to 10 cm².

The conductive material superimposes at least a part of the sensor surface. The conductive material can comprise any material that is able to conduct an electrical current. The conductive material can comprise at least one conducting material selected from the group consisting of a metal, a semimetal, a conducting polymer and a conducting inorganic material or a mixture of at least two thereof.

The metal can preferably be selected from the group consisting of gold, silver, platinum, tungsten and palladium or a mixture of at least two thereof. Preferably, the conductive material comprises gold.

The semimetal can preferably be selected from the group consisting of silicium, boron, alpha tin or a mixture of at least two thereof.

The conducting polymer can preferably comprise a polymer selected from the group consisting of a polythiophene, a polypyrrole, a polyaniline, a polyacetylene, a polyisothionaphthalene and a poly-p-phenylene and derivatives and mixtures or copolymers of at least two thereof. Examples of conductive polymers are poly(p-phenylenevinylene), poly(p-phenylene sulphide), poly(3,4-ethylenedioxythiophene) or mixtures of at least two thereof.

The inorganic material can preferably be selected from the group consisting of an electrically conductive ceramic material, a graphene, graphite or a mixture thereof.

The conducting inorganic material can preferably be an electrically conductive ceramic material, also called cermet, which comprises a ceramic and a metallic material. The ceramic material can preferably comprise one or more elements selected from the group consisting of oxygen, carbon, boron, nitrogen, silicon or a mixture of at least two thereof. The ceramic material can be selected from the group consisting of aluminium oxide, particularly Al₂O₃; zirconium oxide, particularly ZrO₂; manganese oxide, particularly MgO; zirconium toughened aluminium oxide (ZTA); aluminium nitride; aluminium titanate; a piezoceramic selected from the group consisting of Ba (Zr, Ti)O₃, Ba (Ce, Ti)O₃, potassium sodium niobate (PSN), PSN-LiSbO₃ and PSN-LiTaO₃ or a mixture of at least two thereof. The metal material of the conducting inorganic material can preferably comprise a metal selected from the group consisting of nickel, molybdenum, a molybdenum alloy, iridium, tantalum, a tantalum alloy, cobalt, a cobalt-chromium alloy, titanium, a titanium alloy, niobium, a niobium alloy, platinum, a platinum alloy, tungsten, a tungsten alloy, steel or a mixture of at least two thereof. The electrically conductive ceramic can be selected from the group consisting of tungsten silicide (WSi₂), tungsten carbide (WC), titanium nitride (TiN), titanium carbonitride (TiCN), titanium carbide (TiC).

The conductive material can comprise the conducting material in a range of from 10 to 100 wt.- %, or in a range of from 20 to 100 wt.-%, or in a range of from 30 to 95 wt.-% based on the total weight of the conductive material. Preferably, the conductive material comprises gold in a range of from 50 to 100 wt.-%, or in a range of from 70 to 100 wt.-%, or in a range of from 80 to 100 wt.-%.

The conductive material superimposes at least a part of the sensor surface of the substrate. The conductive material can superimpose the sensor surface in a range of from 5 to 100 %, or in a range of from 10 to 95 %, or in a range of from 20 to 90 % by area. The conductive material can superimpose an area of the surface of the substrate in a range of from 0.01 mm² to 50 cm², or in a range of from 0.1 mm² to 40 cm², or in a range of from 0.5 mm² to 10 cm².

Superimposing of the sensor surface by the conductive material can be achieved by all methods the person skilled in the art would choose for this purpose. Superimposing can be achieved for example by one or more deposition techniques, such as one or more deposition techniques selected from the group consisting of printing, spin coating, dispensing, doctor blading, spraying, dripping, laying, coating, impregnating or dipping or a combination of at least two thereof. The printing can preferably be selected from the group consisting of offset printing, gravure printing, inkjet printing, screen printing, stencil printing, tampon printing, flexo printing, or a combination thereof. The coating can preferably be selected from the group consisting of a chemical vapor deposition, a physical vapor deposition, a chemical and electrochemical coating, a spraying, an optical coating or a combination of at least two thereof. The physical vapor deposition can be selected from the group consisting of a cathodic arc deposition, an electron beam physical vapor deposition (EBPVD), an ion plating, an ion beam assisted deposition (IBAD), a magnetron sputtering, a pulsed laser deposition, a sputter deposition, a vacuum deposition, a evaporation deposition like a vacuum evaporation, or a combination of at least two thereof. Preferably, the superimposing is realized by a sputtering process of gold onto the substrate. By superimposing at least a part of the sensor surface by the conductive material one or more conductive contact leads can be achieved. Preferably, more than one conductive contact lead is provided on the sensor surface. Preferably, three conductive contact leads are applied to the sensor surface by the superimposing process. The conductive contact leads can be separated from each other by a width in a range of from 0.1 to 10 mm, or in a range of from 0.3 to 5 mm, or in a range of from 0.5 to 2 mm. Each contact lead can build the basis for an electrode. As outlined above, the sensor may preferably comprise at least two electrodes, wherein at least one working electrode is provided, the working electrode having the conductive material and the first electrode material. Additionally, as outlined above, one or more further electrodes may be provided, such as one or more reference electrodes and/or one or more counter electrodes. The one or more further electrodes each may comprise at least one conductive material and/or at least one contact lead and, additionally or optionally, at least one further electrodes material, such as at least one reference material. Additionally, the at least one working electrode and, optionally, the at least one further electrodes, may fully or partially be covered by at least one protective layer, such as a membrane, which is permeable for the analyte to be detected and/or for at least one electrolyte of the sample, whereas the first electrode material may be retained and may be prevented from contacting the sample and/or a surrounding body tissue. Thereby, a biocompatibility of the sensor may be provided. As an example, reference may be made to WO 2007/071562 A1.

The first electrode material forms a working electrode or at least part of a working electrode.

Besides the detection material, the first electrode material can comprise at least one material that is able to conduct an electrical current. The material that is able to conduct an electrical current is called a conducting material. The conducting material may preferably be selected from the group consisting of a metal, a semimetal, a conducting polymer or a mixture of at least two thereof.

The metal may preferably be selected from the group consisting of gold, silver, platinum, tungsten, palladium or a mixture of at least two thereof.

The semimetal can preferably be selected from the group consisting of silicium, boron, alpha tin or a mixture of at least two thereof.

The conducting polymer may preferably comprise a polymer selected from the group consisting of a polythiophene, a polypyrrole, a polyaniline, a polyacetylene, a polyisothionaphthalene and a poly-p-phenylene and derivatives and mixtures or copolymers of at least two thereof. Examples of conductive polymers are poly(p-phenylenevinylene), poly(p-phenylene sulphide), poly(3,4-ethylenedioxythiophene).

The inorganic material may preferably be selected from the group consisting of an electrically conductive ceramic material, a graphene, graphite or a mixture thereof. Preferably, the conductive material comprises graphite.

The analyte can be any compound or element that is of interest to be detected. The analyte can be part of a sample. The sample normally is a fluid or liquid or a mixture thereof. In the medical field, for example, the analyte can be a compound in a sample like a tissue or a body fluid of a patient. The body fluid may preferably be selected from the group consisting of blood, serum, plasma, interstitial fluid, urine, saliva, sweat or a combination of at least two thereof. The analyte may preferably be selected from the group consisting of glucose, lactose, lactate, uric acid, urea, cholesterol, triglyceride and any other compound in a body fluid of a patient or a combination of at least two thereof. In the following, without intending to restrict the scope of the invention, mainly reference will be made to the electrochemical detection of glucose in blood and/or interstitial fluid.

The sample can be applied at least to the working electrode of the sensor either in vitro or in vivo. The sample for the in vitro analysis can have a volume in a range of 0.001 to 10 ml, or in a range of 0.01 to 5 ml, or in a range of from 0.05 to 1 ml. The sample for the in vitro analysis can be applied by any means suitable for this purpose. Application of the sample can be selected from the group consisting of pipetting, dipping, dosing and a mixture of at least two thereof. For the in vivo analysis, at least the working electrode can be brought into contact with a body fluid of a patient.

Thus, preferably, the sensor may be an implantable sensor, which at least partially is implantable into a body tissue of a user. The touching can be a fixation, for example by adhesion, or a contacting of the sensor at least with the working electrode to a region of the body where the working electrode can come into contact with a body fluid, such as sweat or saliva. Preferably, the sensor is introduced under the skin into the interstitial fluid. Therefore, the sensor preferably is biocompatible.

Furthermore, the first electrode material comprises Mn₂O₃ particles. The Mn₂O₃ particles can catalyze at least a part of the detection reaction of the analyte or a product of the analyte in a first detection reaction with the detection material. For example, a part of the detection reaction can be an electrolysis reaction, achieved according to equation (I):

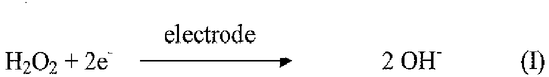

The reduction reaction of H₂O₂ shown in equation (I) is catalyzed by the Mn₂O₃ particles. It has been found that by using Mn₂O₃ particles the reduction reaction of H₂O₂ can be achieved in a voltage range, where a reduction of O₂ does not occur. Thus, the resulting signal is not falsified by a reaction of oxygen at any of the electrodes. The detection reaction, in the form of the reduction of H₂O₂. can be achieved at the working electrode if a sufficient voltage is applied between the working electrode and the reference and/or counter electrode. This voltage is also called decomposition voltage. For an electrochemical sensor, also referred to as an electrolytic sensor, the decomposition voltage of the equation (I) should be different from the decomposition voltage of the reaction of oxygen (O₂), in form of a reduction, at the working electrode. The decomposition voltage of a component at the working electrode can amongst other things depend on the material the working electrode comprises. By adding Mn₂O₃ particles to the first electrode material the decomposition voltage of H₂O₂ can be shifted to a positive potential range. The voltage applied to the working electrode can be selected in a range of from 0.025 to 0.175 V, or in a range of from 0.050 to 0.150 V, or in a range of from 0.100 to 0.130 V if compared to a reference electrode comprising Ag/AgCl with 3 M KCl. One positive effect of choosing this range of voltage is the fact that the reaction of H₂O₂ at the working electrode is specific. That means that, preferably, almost no other component of the sample is oxidized or reduced at the working electrode. By shifting the decomposition voltage to lower voltages, also further reactants can be reduced at the working electrode, such as oxygen as mentioned before.

The first electrode material superimposing at least a part of the conductive material can also mean that a part of the substrate is also superimposed by the first electrode material. Superimposing at least a part of the conductive material by the first electrode material can be achieved by printing, layering, coating, impregnating or dipping or a combination thereof, preferably by printing. At least printing is a form of superimposing at least a part of a surface, wherein the first electrode material is applied as a liquid phase and/or as another form of a deformable material, such as a paste. By printing, the liquid phase and/or paste is applied via an aid onto the sensor surface of the substrate. This can be achieved by different forms of aids. The liquid phase and/or paste can, for example, be applied via a nozzle or valve by extruding or spraying. Alternatively or additionally, the liquid phase and/or paste can be applied or printed via a roll or a drum. As printing methods, gravure printing via a roll or ink-jet printing through an opening, e.g. a nozzle or valve, as well as screen printing through a mesh and spin coating are well known. During the superimposing process, pressure can be applied to the liquid phase and/or paste or the substrate. Alternatively, the liquid phase and/or paste is applied using gravity alone.

The nozzle or valve can operate by a piezo element or a pneumatic valve as they are often used for ink-jet printers. These valves have the property of building portions of the applied liquid phase and/or paste that might preferably be applied under pressure to the surface. The portions of the liquid phase and/or paste preferably have a volume in a range of from 0.1 to 500 nl, or preferably in a range of from 1 to 100 nl, or preferably in a range of from 1 0 to 50 nl.

In the gravure printing process, the surface to be superimposed is fed between two rolls which are in contact with each other. One roll is called the impression roll and the other roll is called the gravure roll because the liquid phase and/or paste comes into contact with it. By guiding the substrate between the contacting rolls with the sensor surface facing towards the gravure roll, the liquid phase and/or paste can be transferred to the sensor surface of the substrate.

In the screen printing process, typically, a paste is forced through a mesh onto the surface of the substrate. This can be achieved by gravity alone or alternatively or additional by using a squeegee or doctor knife. The described superimposing processes can be repeated several times to superimpose more than one part of the sensor surface.

The liquid phase or paste can comprise any liquid or solid matter the person skilled in the art would use to carry the first electrode material. Preferably, the liquid phase comprises a liquid that is able to dissolve at least a part of the first electrode material. The liquid phase or paste preferably comprises at least one compound selected from the group consisting of an organic compound and an inorganic compound, preferably water; or mixtures thereof. In the case of the liquid phase and/or paste comprising an organic compound, the organic compound can be selected from the group consisting of an alcohol, an amine, an ester, an ether, a hydrocarbon, a sulfoxide, a sulfone, a sulfonate, a lactone, a lactam, a nitro compound, a nitrile and an oil or a combination of at least two thereof. Furthermore, the liquid phase and/or paste can comprise an organic compound selected from the group consisting of an aliphatic alcohol, an aromatic alcohol, a cyclic alkene alcohol, a glycol ether, an aliphatic hydrocarbon, an aromatic hydrocarbon, a branched hydrocarbon, a benzene, a halogenated hydrocarbon, a glycol ether acetate or a combination of at least two thereof. The organic compound of the liquid phase and/or paste is preferably selected from the group consisting of methanol, ethanol, butanol, propanol, acetone, γ-butyrolactone, N-methyl-2-pyrrolidone, acetonitrile, nitromethane, triethylamine, dimethylformamide, heptane, hexane, dimethylsulfoxide, sulfolane, ethylene carbonate, ethylene glycol monobutyl ether, dimethylcarbonate, propyleneglycol methylether acetate, propyleneglycol methylether acetate or a mixture of at least two thereof. The liquid phase and/or paste can comprise an organic compound in a range of from 0.1 to 99 wt.-%, or preferably in a range of from 1 to 95 wt.-%, or preferably in a range of from 10 to 90 wt.-%, or preferably in a range of from 20 to 80 wt.-%, each based on the total weight of the liquid phase and/or paste.

The liquid phase and/or paste generally may comprise one or more solvents and/or binders. Therein, inorganic and/or organic solvents may be used.

In the case where the liquid phase and/or paste comprises an inorganic compound, the inorganic compound can be selected from the group consisting of water, an acid and a base, especially hydrochloric acid, nitric acid, sulfuric acid and alkaline lye or mixtures thereof. The liquid phase and/or paste can comprise an inorganic compound in a range of from 0.1 to 99 wt.-%, or in a range of from 1 to 95 wt.-%, or in a range of from 10 to 90 wt.-% each based on the weight of the liquid phase and/or paste. The mentioned components for the liquid phase and/or paste add up to 100 wt.-%.

The liquid phase and/or paste can have a viscosity in a range of from 100 to 50000 mPa^{∗}s, or in a range of from 500 to 10000 mPa^{∗}s, or in a range of from 1000 to 5000 mPa^{∗}s.

After superimposing the sensor surface by the liquid phase and/or paste, comprising at least the first electrode material, the liquid phase and/or paste is dried to form the working electrode. Drying can be established by any method that is suitable for the purpose of the invention. The drying can be supported by different drying methods. The drying methods can be selected from the group consisting of a heating, a blowing, an irradiation or a combination of at least two thereof. The heating can be achieved by the provision of a heating oven or by the supply of a heated surface or by a combination thereof. The blowing can be achieved by the supply of a gas flow, for example by heated air. The irradiation can be achieved by a UV or an IR lamp. The drying can be achieved at a temperature in a range of from 20 to 40 °C, or in a range of from 25 to 35 °C. The drying is provided for a time period in a range of from 1 minute to 24 hours, or in a range of 30 minutes to 10 hours, or in a range of 1 hour to 5 hours. By drying the liquid phase and/or paste on the substrate, the working electrode is provided.

With these application or superimposing methods it is possible to create a pattern of the first electrode material onto the surface of the substrate. At least one dot, line or grid of the first electrode material can be formed by the superimposing process. The at least one dot or line can have a diameter or width in a range of from 0.01 to 10 mm, or in a range of from 0.05 to 5 mm, or in a range of from 0.1 to 1 mm. The lines of the grid can lie in the same ranges as mentioned for the dots. The dots or the lines of the grid can have a distance or mesh size in a range of from 0.05 to 10 mm, or in a range of from 0.1 to 5 mm, or in a range of from 0.5 to 3 mm. The at least one dot, line or grid formed of first electrode material can form the at least one working electrode or a part thereof. The dots or lines in a grid can have the same or differing extensions, especially the same or different width and length. The length of the lines of the grid can lie in a range of from 0.01 to 50 mm, or in a range of from 0.05 to 10 mm, or in a range of from 0.1 to 5 mm. The height of the dots or lines can be in a range from 0.01 to 10 mm, or in a range of from 0.1 to 5 mm, or in a range of from 0.5 to 2 mm.

The working electrode forms at least one working electrode pad, wherein the sample is directly or indirectly applicable to the working electrode pad. Each working electrode pad is preferably formed by at least one dot, line or grid which each can form a coherent area of the first electrode material. If more than one dot, line or grid of the first electrode material is superimposed on the conductive material, the sensor provides more than one electrode pad. All electrode pads together build the working electrode. The sensor can comprise a working electrode with a number of electrode pads in a range of from 1 to 50, or in a range of 2 to 30, or in a range of 5 to 20 electrode pads.

As outlined above, specifically in the case that the sensor is implantable into a body tissue of a user, the sensor may fully or partially be covered by at least one protective layer, such as a biocompatible membrane, such as a polymethacrylate membrane, specifically a membrane covering the at least one working electrode pad.

Furthermore an embodiment of the sensor is provided, wherein the conductive material of the working electrode pad is electrically connected to at least one electrically conductive contact lead.

A further embodiment of the sensor is provided, wherein the first electrode material comprises Mn₂O₃ particles in a range of from 1 to 90 wt.-%, or in a range of from 3 to 80 wt.-% or in a range of from 5 to 50 wt.-% based on the total weight of the first electrode material, preferably in a fully dried state.

The oxidase is selected from the group consisting of an alcohol oxidase, a glucose oxidase, an uricase, monoamine oxidase, cytochrome P450 oxidase, NADPH oxidase, Xanthine oxidase, L-gulonolactone oxidase, laccase, lysyl oxidase or a mixture of at least two thereof. In a preferred embodiment of the sensor the enzyme is glucose oxidase (GOD).

The first electrode material is adapted such that H₂O₂ is generated in the detection reaction. When providing the sensor as an electrochemical sensor, the H₂O₂ can be reduced at the working electrode to generate an electrical current that can be detected. The H₂O₂ is preferably formed in the detection reaction of the analyte with the detection material in a stoichiometric relation. Stoichiometric means in the sense of the invention, that the detection reaction of one analyte molecule delivers a natural number of H₂O₂ molecules or that the H₂O₂ molecule that is built during the detection reaction is built from one or more analyte molecules. Preferably, in the detection reaction one, two or more H₂O₂ molecules are formed during the reaction of one analyte molecule. For example, during the reaction of one molecule glucose with the enzyme GOD in an aqueous surrounding one H₂O₂ is generated according to the equation II:

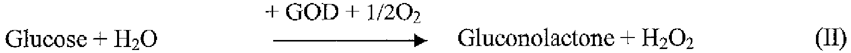

According to equation (I), as described above, the H₂O₂ molecule can be oxidized at the working electrode.

To achieve a sensor with a low resistance and therefore a low energy demand, the components of the sensor should be selected appropriately. During the reaction according to equation (I) a current occurs or may be measured when closing an appropriate electric circuit. The current should be conducted from the working electrode to the detection device. This can be achieved by the connection of the first electrode material with the conductive material. The conductive material should be able to conduct an electrical current and to be electrically connectable to a detection device. The detection device may comprise at least one current measurement device and/or at least one voltage measurement device, such as a potentiometer or potentiostat. Examples of an appropriate measurement setup including a detection device are given in WO 2007/071562 A1. Other detection devices are feasible.

The conducting material comprises a component selected from the group consisting of gold, platinum, palladium, silver or a mixture of at least two thereof. The conducting material can form the contacting leads. The contacting leads can have a thickness in a range of from 10 to 250 µm, or in a range of from 30 to 180 µm, or in a range of from 50 to 150 µm.

At least a part of the sensor is superimposed by a protective layer. Normally, the protective layer is utilized in a biosensor that is introduced into the body, such as a body tissue and/or a blood vessel, of a user, such as a patient. The function of the protective layer may be to convert the sensor into a biocompatible device. Preferably, the protective layer may be or may comprise at least one membrane. The protective layer can be the layer that comes into contact with parts of a body, like interstitial fluid, blood or tissue of a patient when the sensor is implemented into the body of the patient. The protective layer can serve as a biocompatible layer. Therefore the protective layer can comprise at least partly a biocompatible material. The protective layer can comprise a biocompatible material in a range of from 50 to 100 wt.-%, or in a range of from 70 to 100 wt.-%, or in a range of from 90 to 100 wt.-% based on the total weight of the protective layer. A biocompatible material is a material that does not affect a living body negatively when coming into contact with the living body. To determine whether a material is biocompatible the material can be used in accordance with ISO 10993. The protective layer can comprise a polymer and a protein or a mixture thereof. The polymer can be selected from the group consisting of a polymethacrylate, a polyurethane (PU), a polyvinyl alcohol (PVA), a polyimide and a cuprophane (CUP) or a mixture of at least two thereof. Preferably, the protective layer comprises poly(2-methacryloyloxyethyl phosphorylcholine-co-n-butyl methacrylate) (MPC). The MPC may be solved in ethanol.

The substrate comprises a biocompatible material. The biocompatible material can be the same as mentioned in the context of the protective layer. The substrate can comprise the biocompatible layer in a range of from 1 to 100 wt.- %, or in a range of from 30 to 100 wt.-%, or in a range of from 50 to 100 wt.-%.

The sensor comprises at least one further electrode selected from the group consisting of a reference electrode, a counter electrode and a combined reference and counter electrode. Thus, as an example, the sensor may be a sensor of a two-electrode setup, comprising at least one working electrode and at least one combined counter/reference electrode, or the sensor may be a sensor of a three-electrode setup, comprising at least one working electrode, at least one counter electrode and at least one reference electrode being separate from the counter electrode.

The further electrode comprises a further electrode material. The counter electrode may comprise at least one electrode material, preferably a conductive electrode material such as a metal. The metal can be selected from the group consisting of Au, Ag, Pd, Pt or a mixture of at least two thereof. The reference electrode can comprise a further electrode material selected from the group consisting of a metal, a metal sulfate and a metal halide or a mixture thereof. The metal can be selected from the group consisting of Au, Ni, Ag, Hg, Cu, Pd, Pt or a mixture of at least two thereof. The metal halide can be selected from the group consisting of AgCl, AgBr, AgI, Hg₂Cl₂, CuSO₄ or a mixture thereof. The reference electrode can comprise a mixture of Ag and AgCl in a range of from 1 to 100 wt.-%, or in a range of from 30 to 100 wt.-%, or in a range of from 50 to 100 wt.-% based on the total weight of the reference electrode. The counter electrode can comprise the same components as the working electrode, beside the detection material. For example the counter electrode can comprise graphite. The counter electrode can have a size, where an additional reference electrode is dispensable. For example, the counter electrode has a size for which the potential does not change more than 10 mV during an operation of the sensor measuring currents.

### EXAMPLES:

### 1. Preparation of working electrodes

To prepare a working electrode on the substrate of the sensor a liquid phase and/or paste is superimposed on a part of the conductive material which superimposes at least a part of the sensor surface on the substrate. The liquid phase and/or paste in the form of a paste for forming a working electrode comprises the following components:
- 66.80 wt.-% graphite PE401 from Acheson Industries Deutschland (Germany);
- 0.8 wt.-% glucose oxidase from Roche Diagnostics GmbH, Germany;
- 15.4 wt.-% Mn₂O₃ particles from Strem Chemicals Inc., Germany;
- 17 wt.-% DEGMBE, from Merck KGaA, Germany.

The graphite PE401 comprises:
- 36 wt.-% graphite;
- 64 wt.-% unknown solvent

The components are mixed twice for 5 minutes in a speed-mixer (Hauschild & Co KG, Germany) at 3000 rpm . The mixture is applied to the sensor surface to superimpose at least a part of the conductive material. The mixture is vacuum dried for 240 minutes at room temperature

### 2. Preparation of reference electrodes

To prepare a reference electrode on the substrate of the sensor a liquid phase and/or paste is superimposed on a part of the conductive material which superimposes at least a part of the sensor surface on the substrate. The liquid phase and/or paste for forming a reference electrode comprises the following components:
- 94 wt.-% Electrodag 6037SS from Acheson Industries Deutschland (Germany);
- 6 wt.-% DEGMBE from Merck KGaA, Germany

The components are mixed 4 times for 15 seconds in a speed-mixer (Hauschild*&* Co KG, Germany) at 3000 rpm. The mixture is applied to the sensor surface to superimpose at least a part of the conductive material. The mixture is dried for 120 minutes at 80 °C at normal pressure.

### 3. Preparation of counter electrodes

To prepare a counter electrode on the substrate of the sensor a liquid phase and/or paste is superimposed on a part of the conductive material which superimposes at least a part of the sensor surface on the substrate. The liquid phase and/or paste for forming a counter electrode comprises the following components:
- 94 wt.-% graphite PE401 from Acheson Industries Deutschland (Germany);
- 6 wt.-% DEGMBE from Merck KGaA, Germany

The graphite PE401 comprises:
- 36 wt.-% graphite;
- 64 wt.-% unknown solvent

The components are mixed for 5 minutes in a speed-mixer (Hauschild& Co KG, Germany) at 3000 rpm. The mixture is applied to the sensor surface to superimpose at least a part of the conductive material. The mixture is dried for 120 minutes at 80 °C.

### Description of Figures:

Further optional features and embodiments of the invention will be disclosed in more detail in the subsequent description of preferred embodiments. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1a:: shows a schematic top view of a sensor not according to the invention with a working, a reference and a counter electrode;
- Figure 1b: shows a schematic side view of a sensor not according to the invention with a working, a reference and a counter electrode;
- Figure 2:: shows a diagram of measurement results of two electrodes, one with and one without Mn₂O₃;
- Figure 3:: shows a diagram of measurement results of a sensor with Mn₂O₃-GOD-Graphite matrix for different glucose concentrations;
- Figure 4:: shows a block diagram of results of a sensor with Mn₂O₃-GOD-Graphite matrix in 10 mM glucose solution for different interference molecules;
- Figure 5: shows a schematic view of a sensor system not according to the present invention, the sensor system comprising a detecting device;
- Figure 6: shows a scheme of a production process for a sensor not according to the invention.

### Detailed description of the Figures

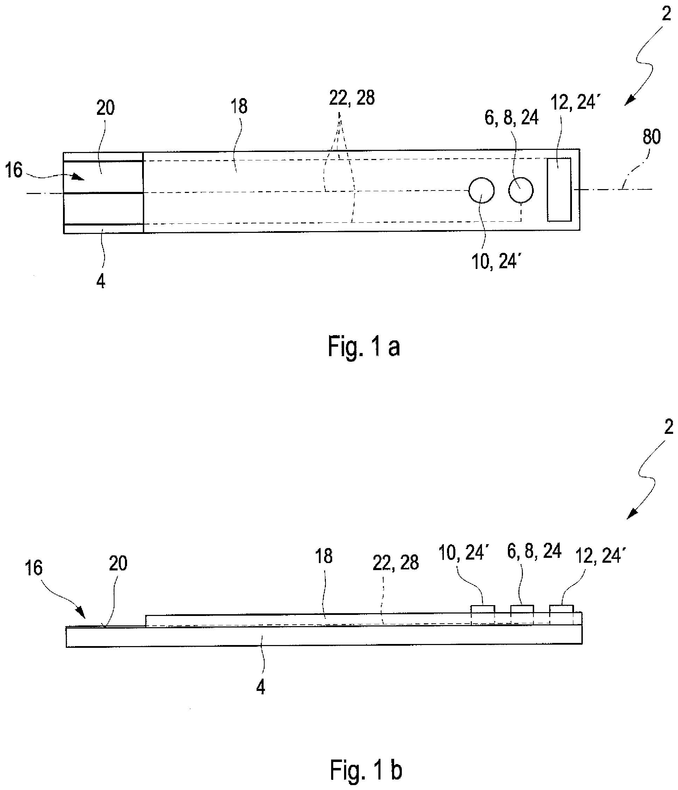

In Figure 1a schematic top view of a sensor 2 not according to the invention is shown. The sensor 2 comprises a substrate 4 made from a Melinex^{®} foil from DuPont Teijin Films, Europe. The sensor 2 has a rectangular shape; however the sensor 2 can have any form that is suitable to detect an analyte in a sample 26. In this example, the sensor 2 has a length of 2 cm, a width of 0.5 mm and a height of 0.15 mm in the contacting region 16. The height of the rest of the sensor 2 might be greater than 0.15 mm as the electrodes 6, 10, 12 and/or the isolation layer 18 may be thicker in this region of the sensor 2. The height of the sensor in the region outside of the contacting region may be in the range from 0.15 to 2 mm.

On a sensor surface 20 of the substrate 4, three conductive contact leads 28 are positioned next to each other with a distance of 0.05 mm each. The width of each conductive contact lead 28 is 0.05 mm. The conductive contact leads 28 build three parallel lines made of gold. At one end of the substrate 4, in figure 1 on the left hand side, a contacting region 16 is positioned. The starting points of the conductive contact leads 28 build a line perpendicular to the left boarder of the substrate 4. The length of each conductive contact lead 28 can vary depending on the electrode they are contacted with. In this example the conductive contact lead 28 in the middle of the sensor 2 is the shortest as it is contacted to the reference electrode 10. The conductive contact leads 28 have a length in a range of 1.5 to 1.9 cm. The longest conductive lead 28 is that contacted to the counter electrode 12. The conductive contact lead 28 contacted with the working electrode 6 provides a curve at the right end of the substrate 4 to reach that the first electrode material 24 can be positioned on the center axis 80 of the sensor 2.

The substrate 4 can expand its width in the contacting region 16 of the sensor 2. The contacting region 16 can have a dimension of 0.5 cm in length, 4.5 mm in width and 0.15 mm in height. Thus, the width of the sensor 2 is broadened in this part of the sensor 2. The broadened contacting region 16 of the substrate 4 can be positioned asymmetrically, only on one side of the sensor 2 or symmetrically on both sides of the sensor 2.

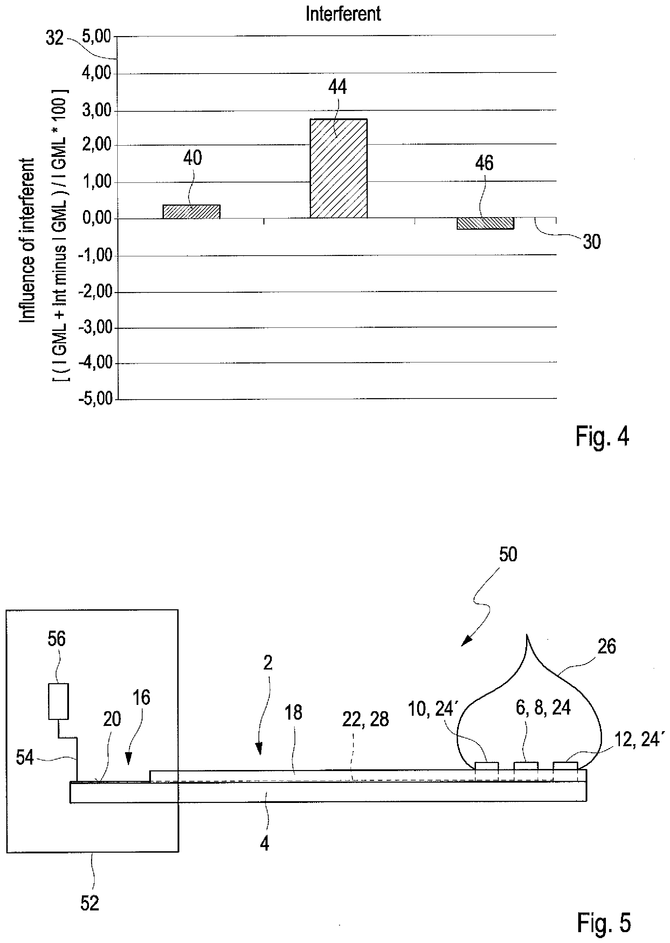

In the contacting region 16 also the conductive contact leads 28 might be broader than on the rest of the substrate 4. In the contacting region 16 the conductive contact leads 28 have a width of 1 mm over a length of 0.5 cm. The height stays the same as mentioned above. In the contacting region 16 of the sensor 2 the conductive contact leads 28 end. This is the region where a detection device 52 can be connected with the sensor 2 as shown in figure 5. On the three conductive leads 28, three different electrode materials 24, 24' are positioned. The working electrode 6 comprises a first electrode material 24, in the form of a graphite matrix with glucose oxidase and Mn₂O₃. The reference electrode 10 comprises a further electrode material 24', in form of an Ag/AgCl and the counter electrode 12 comprises a further electrode material as described in the examples.

An isolation layer 18 covers the working electrode 6, the reference electrode 10 and the counter electrode 12. The isolation layer 18 can also cover most of the substrate 4 and the conductive contact leads 28. The height of the isolation layer 18 is in the range of from 0.01 to 0.02 mm. However, one end of each of the conductive contact leads 28 is not covered by the isolation layer 18. Alternatively the isolation layer provides at least one hole for contacting the contact leads 28. This is the part of the sensor 2 that can be contacted by the detection device 52. The position of the electrodes 6, 10, 12 can vary. It is not obligatory that the working electrode 6 is positioned in the middle of the three electrodes 6, 10, 12. It is also possible to position the reference electrode 10 or the counter electrode 12 in the middle of the substrate 4. If the sensor 2 was dip coated to cover it with a protective layer, the surface opposite to the illustrated sensor surface 20 is also covered by the protective layer (not shown here).

In figure 1b the sensor 2 is shown as a side view. Compared to the top view of figure 1a the sensor 2 shown in figure 1a is rotated about the dotted line 80 in the middle of the sensor 2 for 90 °. In this side view the sequence of the materials of the sensor 2 is illustrated. The sensor 2 of figure 1b shows a layered structure. At the bottom of the sensor 2 the substrate 4 builds the first layer as base of the sensor 2.

On the substrate 2 a layer of a conductive contact lead 28 is positioned. On the conductive contact leads 28 an isolation layer 18 is positioned, beside the contact region 16 and the part of the conductive material 22 where the first electrode material 24 is positioned to build the working electrode 6, a further electrode material 24' to build the reference electrode 10 and a further electrode material to build the counter electrode 12. Not shown in the side view is the detail that the conductive contact lead 28 is connected to the further electrode material 24' of the reference electrode 10.

There might be provided a further protective layer, such as a diffusion membrane, on the whole sensor 2 beside the contacting region 16. Furthermore, a protective layer may cover the sensor 2 beside the contacting region 16. The protective layer can be a biocompatible material when the sensor should be at least partly introduced into a human body.

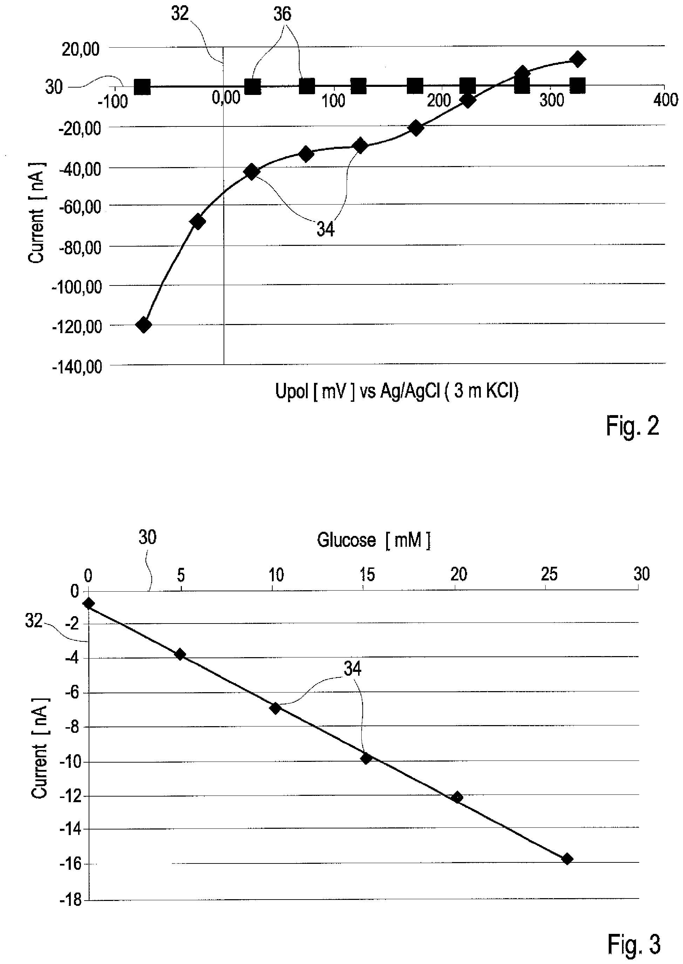

Figure 2 shows a diagram of measurement values 34, 36 of two sensors at different polarization voltages at a constant concentration of H₂O₂ as analyte. One of the sensors comprised a working electrode 6 of PE401 (Acheson, see examples) and is symbolized by the squares 36. The other sensor comprised a working electrode 6 with Mn₂O₃ comprised 84,6 wt.-% (Acheson PE401), 15,4 wt.-% Mn₂O₃, symbolized by the rhombs 34. The counter electrodes 12 of both sensors comprised 100 wt.-% of Acheson PE401. The reference electrode 10 of both sensors comprised Electrodag 6037SS (Acheson). The two sensors with and without Mn₂O₃ resulting in measurement values 34 and 36 both comprised a similar composition of the counter electrodes 12.

For the measurement shown in Figure 2, the two sensors were independently positioned in a 10 mM phosphate buffer pH 7.4 with 147 mM NaCl and 50 µM H₂O₂ in a way that the electrodes 6, 10, 12 are dipped into the buffer solution.

The buffer solution was prepared by solving the mentioned salts in deionized water by Milli-Q Academic (Merck Millipore) for 30 minutes at a temperature of 37 °C before adding the H₂O₂. The sensors were dipped into the so prepared buffer solution.

The sensors were contacted by the contacting region to a Gamry potentiostat G300 from Gamry Instruments (USA). The power supply was regulated to supply 325 mV (vs Ag/AgCl 3M KCl) between the working and the reference electrode at the start of the measurement. This voltage was fixed for 10 minutes. After that period the voltage was changed for eight times to a further voltage 50 mV less than the foregoing voltage ending at -75 mV. After each change of the voltage a measurement of the current was established for 10 minutes. The current was measured by the detection device. Results for the sensor comprising Mn₂O₃ and for the sensor without Mn₂O₃ are shown in Figure 2.

Comparison of the measurement values 34 of the sensor with Mn₂O₃ and those of the sensor without Mn₂O₃ illustrated as points 36, shown in Figure 2, show that the graphite electrode 10 of sensor without Mn₂O₃ with measurement values 36 does not deliver a current flow when dipped into a solution comprising H₂O₂, independent of the polarization voltage applied in a range of from -75 mV to 325 mV. However, the sensor with Mn₂O₃ represented by measurement values 34 shows a change of current starting from 325 mV to about 175 mV. From 175 mV to 25 mV the sensor with Mn₂O₃ delivers a constant current in a range of -25 to 40 nA. Not before the decrease of the voltage to 25 mV and less, the sensor with Mn₂O₃ delivers stable current results. The increase of the current beyond the 25 mV to negative voltages, result in a reaction of the electrode with oxygen O₂ included in the solution. This is a remarkable result as the electrodes with different metal comprising catalysts deliver a reaction with H₂O₂ at lower voltages. It is an astonishing result that a sensor could be run with such low voltages without interference of the oxygen reaction.

Figure 3 shows a diagram of a measurement of a sensor 2 with a working electrode 6 comprising graphite, GOD and Mn₂O₃, a reference electrode 10 and a counter electrode 12, all prepared as described in the example section above. The sensor 2 was dipped into a buffer solution as prepared for the experiments of Figure 2 without the addition of H₂O₂. The setup of the measuring system was the same as for the experiments of Figure 2. At a voltage of 125 mV between the working electrode 6 and the reference electrode 10, the current was measured by the detection device. After 25 minute equilibration a current of 0.8 nA was measured. After this measurement the sensor 2 was dipped into a buffer solution as described before with 5 mM Glucose. After 25 minute equilibration the current was measured again. The current changed to 3.8 nA, as the measurement values 34 show. This was repeated for a concentration of 10, 15, 20 and 25 mM Glucose in the same was as described before. The measurement values 34 of Figure 3 show a linear increase of the current by an increase of the glucose concentration. A disturbance of oxygen during these measurements was not measured.

In Figure 4 the influence on the measurement results of a sensor as described for Figure 3 by adding different interference materials are shown. At a voltage of 125 mV between the working electrode 6 and the reference electrode 10, the current was measured by the detection device. The first block 40 represents the percentage of interference with the signal when 2 mg/dl ascorbic acid was added to a buffer solution as described for the measurements in Figure 2 and 3, containing glucose with a concentration of 10 mM. After 25 minute equilibration the current was measured. The same buffer was used for the interference measurements of second block 44 representing the disturbance of uric acid with a concentration of 8 mg/dl. A third interfering material was acetaminophen in a concentration of 3 mg/dl represented by the third block 46 of the block diagram.

As can be seen from the diagram the interference of uric acid is the greatest. Here an increase of the signal is provoked by adding uric acid to the sample solution. The acetaminophen has a decreasing effect on the signal of the sensor. The disturbance of ascorbic acid and acetaminophen is in the range of the normal error of measurement. As all three interfering substances show very low signal interference, the measured error of the sensor has been diminished compared to state of the art sensors.

In Figure 5 a sensor system 50 not according to the present invention is shown. A sensor 2, with the same geometry and components as described in Figure 1a and 1b, is positioned with its contacting region 16 into a detector device 52. The detector device 52 provides at least one contact 54 for contacting the conductive contact leads 28 that lead to the working electrode 6, the reference electrode 10 and the counter electrode 12. Preferably, each conductive contact lead 28 is contacted by one contact 54 of the detector device 52. The detector device 52 is able to deliver and keep a constant voltage between the working electrode 6 and the reference electrode 10. By keeping the voltage constant, the measured current between the working electrode 6 and the counter electrode 12 delivers the measurement signal that is proportional to the analyte concentration, when applying the formulas I and II. This current is measured by an electrical detector 56 that measures the current between the electrodes 6 and 12, which is part of the detector device 52.

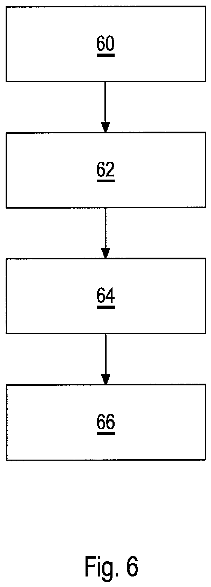

In Figure 6 a scheme of the process for manufacturing a sensor not according to the invention is shown. In a first step P1 60 the substrate 4 is provided, wherein the substrate 4 comprises a sensor surface 20. Providing the substrate in the first step P1 60 is achieved by unrolling a foil of polyimide which is stored on a roll. The foil has a thickness of 0.5 mm. One end of the foil is wound around a second roll. The distance of the rolls is in a range of from 1 to 100 m. In one example the distance is about 10m. The foil has a width of 1 m. The shape of the sensor is embossed about 100 fold per meter in the foil. A conductive material 22 in form of gold is applied to the sensor surface 22 in the second step P2 62 by a sputtering process. The conductive contact leads 28 are formed out of the gold layer by scratching off the superfluous parts of gold. On each sensor surface 20 of the embossed sensors 2 the first electrode material 24 is applied to the conductive contact leads 28 in the third step P3 64 to form a working electrode 6 or a working electrode pad 8. The first electrode material 24 is applied to the conductive contact leads 28 in a way as described in example 1 above. According to a fourth step P4 66, the reference electrode 10 and/or counter electrode 12 are applied to the conductive contact leads 28 as described in examples 2 or 3 above. Optionally a fifth step P5 can be applied (not shown) to cover at least a part of the sensor 2 with a biocompatible membrane and/or a protective layer. This is achieved according example 4 as described above.

### List of reference numerals

- 2: sensor
- 4: substrate
- 6: working electrode
- 8: working electrode pad
- 10: further or reference electrode
- 12: further or counter electrode
- 16: contacting region
- 18: isolation layer
- 20: sensor surface
- 22: conductive material
- 24: first electrode material
- 24': further electrode material
- 26: sample
- 28: conductive contact lead
- 30: x - axis
- 32: y - axis
- 34: measurement values of sensor with Mn₂O₃
- 36: measurement values of sensor without Mn₂O₃
- 40: first block / 2 mg/dl ascorbic acid
- 44: second block / 8 mg/dl uric acid
- 46: third block / 3 mg/dl acetaminophen
- 50: sensor system
- 52: detector device
- 54: contact
- 56: electrical detector
- 60: step 1
- 62: step 2
- 64: step 3
- 66: step 4
- 80: dotted line / center axis of sensor 2

## Claims

1. A process for detecting an analyte that is able to generate H₂O₂ in a sample, the process comprising the steps of:
S1. providing a sensor (2), the sensor (2) comprising:
a. a conductive material (22);
b. a first electrode material (24) superimposing at least a part of the conductive material (22) to form a working electrode (6),
wherein the first electrode material (24) is adapted to perform at least one detection reaction when the analyte is present in the sample,
and wherein H₂O₂ is generated in the detection reaction, wherein the first electrode material comprises Mn₂O₃ particles; and wherein the first electrode material further comprises an enzyme, wherein the enzyme is an oxidase ;
S2. applying a voltage to the working electrode (6);
wherein the voltage is selected from the voltage range at which H₂O₂ in the sample will be reduced at the working electrode (6) thereby generating an electrical current,
wherein the voltage is in a range of from 50 to 150 mV if compared to a reference electrode comprising Ag/AgCl with 3 M KCl,
the process further comprising the detection of the electrical current,
wherein the electrical current corresponds to a measure of a concentration of the analyte in the sample.

## Patentansprüche

1. Verfahren zum Nachweis eines Analyten, der in der Lage ist, H₂O₂ zu erzeugen, in einer Probe, wobei das Verfahren die Schritte umfasst:
S1. Bereitstellen eines Sensors (2), wobei der Sensor (2) umfasst:
a. ein leitfähiges Material (22);
b. ein erstes Elektrodenmaterial (24), welches über mindestens einem Teil des leitfähigen Materials (22) angeordnet ist, um eine Arbeitselektrode (6) zu bilden,
wobei das erste Elektrodenmaterial (24) eingerichtet ist, um mindestens eine Nachweisreaktion durchzuführen, wenn der Analyt in der Probe vorhanden ist,
und in der Nachweisreaktion H₂O₂ erzeugt wird, wobei das erste Elektrodenmaterial Mn₂O₃-Partikel umfasst;
und das erste Elektrodenmaterial des Weiteren ein Enzym umfasst, wobei das Enzym eine Oxidase ist;
S2. Anlegen einer Spannung an die Arbeitselektrode (6);
wobei die Spannung ausgewählt ist aus dem Spannungsbereich, bei dem H₂O₂ in der Probe an der Arbeitselektrode (6) reduziert wird, wodurch ein elektrischer Strom erzeugt wird,
wobei die Spannung in einem Bereich von 50 bis 150 mV liegt, verglichen mit einer Referenzelektrode, die Ag/AgCl mit 3 M KCl umfasst,
wobei das Verfahren des Weiteren den Nachweis des elektrischen Stroms umfasst, wobei der elektrische Strom einem Maß einer Konzentration des Analyten in der Probe entspricht.

## Revendications

1. Procédé pour détecter un analyte qui est susceptible de générer du H₂O₂ dans un échantillon, le procédé comprenant les étapes suivantes :
S1. la fourniture d'un capteur (2), le capteur (2) comprenant :
a. un matériau conducteur (22) ;
b. un premier matériau d'électrode (24) chevauchant au moins une partie du matériau conducteur (22) pour former une électrode de travail (6),
dans lequel le premier matériau d'électrode (24) est conçu pour effectuer au moins une réaction de détection lorsque l'analyte est présent dans l'échantillon,
et dans lequel du H₂O₂ est généré dans la réaction de détection, dans lequel le premier matériau d'électrode comprend des particules de Mn₂O₃ ; et dans lequel le premier matériau d'électrode comprend en outre une enzyme, dans lequel l'enzyme est une oxydase ;
S2. l'application d'une tension à l'électrode de travail (6) ;
dans lequel la tension est choisie dans la plage de tension à laquelle le H₂O₂ dans l'échantillon sera réduit au niveau de l'électrode de travail (6), générant de ce fait un courant électrique,
dans lequel la tension est dans une plage de 50 à 150 mV si on compare à une électrode de référence comprenant Ag/AgCl avec 3 M KCl,
le procédé comprenant en outre la détection du courant électrique,
dans lequel le courant électrique correspond à une mesure d'une concentration de l'analyte dans l'échantillon.
